(19) Europäisches Patentamt
European Patent Office
Office européen des brevets

(11) Numéro de publication: **0 455 917 A1**

# (12) DEMANDE DE BREVET EUROPEEN

(21) Numéro de dépôt: **90401251.5**

(22) Date de dépôt: **11.05.90**

(51) Int. Cl.5: **C07C 231/10**, C07C 233/25, C07C 233/15

(43) Date de publication de la demande: **13.11.91 Bulletin 91/46**

(84) Etats contractants désignés: **AT BE CH DE DK ES GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Metz, François**
**22, rue de Condé**
**F-69002 Lyon(FR)**

(74) Mandataire: **Dutruc-Rosset, Marie-Claude et al**
**RHONE-POULENC CHIMIE Service Brevets Chimie 25, Quai Paul Doumer**
**F-92408 Courbevoie Cédex(FR)**

## (54) Procédé de préparation d'amides n-arylsubstitués.

(57) La présente invention concerne la préparation d'amides N-arylsubstitués par une N-acylation réductrice de composés nitroaromatiques au moyen d'acides carboxyliques et d'oxyde de carbone.

Le procédé selon l'invention est caractérisé en ce que la réaction est conduite en présence d'une quantité catalytiquement efficace de palladium ou d'un composé du palladium et d'au moins un additif de formule :

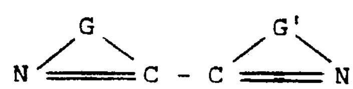

dans laquelle G et G', identiques ou différents représentent chacun un groupe pontant qui comporte 3 ou 4 atomes dont au moins 2 sont des atomes de carbone, G et G' pouvant par ailleurs être reliés l'un à l'autre.

EP 0 455 917 A1

La présente invention concerne un procédé de préparation d'amides N-arylsubstitués. Elle a plus particulièrement trait à la préparation de tels amides par une N-acylation réductrice de composés nitroaromatiques au moyen d'acides carboxyliques et d'oxyde de carbone.

Les amides N-substitués constituent une classe de produits utiles notamment comme intermédiaires de synthèse de diverses substances actives dans les domaines pharmaceutiques et phytosanitaires notamment.

Il a déjà été proposé de préparer des amides N-arylsubstitués par N-acylation réductrice de composés nitroaromatiques.

Ainsi, dans "Bulletin of the Chemical Society of Japan vol 42, 827-828 (1969)" il a été indiqué que le nitrobenzène peut être converti respectivement en acétanilide ou en propionanilide par réaction avec de l'oxyde de carbone et l'acide acétique ou l'acide propionique, à une température supérieure à 300°C et des pressions initiales d'oxyde de carbone mesurées à la température ambiante de 50 à 100 kg/cm2 en présence de nickeltétracarbonyle, de dicobaltoctacarbonyle ou de ferpentacarbonyle. Toutefois le développement d'une telle technique à l'échelle industrielle est largement compromis notamment par les conditions opératoires (température et pression) excessivement sévères requises.

Plus récemment, dans "J. Org. Chem., 1984, 49, 4451-4455 (1984)" il a été proposé une alternative au système catalytique précédemment évoqué, comprenant un complexe du platine tel $PtCl_2(PPh_3)_2$ et du chlorure d'étain (IV) ou un autre acide de Lewis tel $SnCl_2$, $FeCl_3$, $VCl_3$, $AlCl_3$ ou $ZnCl_2$. Si la présence de tels acides de Lewis est indispensable à l'obtention d'une sélectivité convenable en acétanilide, une température de 180°C au moins est nécessaire pour observer une transformation appréciable des composés nitrés et en outre, cette transformation s'accompagne le plus souvent de la co-production d'aniline, non désirable. La présence nécessaire de complexes du platine rares et onéreux compromet également le développement à l'échelle industrielle d'un tel procédé dont l'intérêt de principe n'est pas contesté.

C'est pourquoi il s'avérait nécessaire de rechercher une alternative aux systèmes catalytiques précédemment proposés.

La demanderesse a maintenant trouvé qu'il est possible de préparer des amides N-arylsubstitués par réaction de l'oxyde de carbone et d'au moins un acide carboxylique sur au moins un composé nitroaromatique dans des conditions de pression et de température moins sévères que celles jusqu'alors proposées, tout en limitant la co-production d'amine aromatique à condition de mettre en oeuvre un système catalytique à base de palladium ou d'un composé du palladium et d'au moins un additif dont la nature est spécifiée ci-après.

La présente invention a donc pour objet un procédé de préparation d'amides N-arylsubstitués par réaction en phase liquide, sous pression supérieure à la pression atmosphérique, de l'oxyde de carbone et d'au moins un acide carboxylique sur au moins un composé nitroaromatique caractérisé en ce que la réaction est conduite en présence d'une quantité catalytiquement efficace de palladium ou d'un composé du palladium et d'au moins un additif de formule :

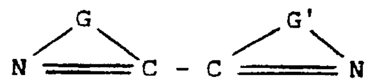

dans laquelle G et G', identiques ou différents représentent chacun un groupe pontant qui comporte 3 ou 4 atomes dont au moins 2 sont des atomes de carbone, G et G' pouvant par ailleurs être reliés l'un à l'autre.

La présente invention a également pour objet plus particulier un procédé de fabrication de formamides N-arylsubstitués par réaction de l'oxyde de carbone et de l'acide formique sur au moins un composé nitroaromatique en présence dudit système catalytique.

Un autre objet plus particulier entrant dans le cadre de la présente invention est un procédé de préparation du p-acétylaminophénol par réaction de l'oxyde de carbone et de l'acide acétique sur le p-nitrophénol en présence dudit système catalytique.

Le procédé selon la présente invention peut être représenté schématiquement par l'équation stoechiométrique suivante :

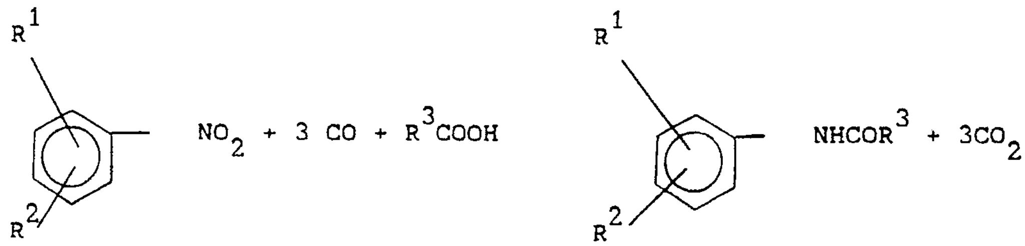

dans laquelle :

- $R^1$ et $R^2$, identiques ou différents, représentent :
  - un atome d'hydrogène,
  - un groupement hydroxyle (-OH),
  - un groupement nitro ($-NO_2$),
  - un atome d'halogène,
  - un radical alkyle, linéaire ou ramifié, renfermant au maximum 12 atomes de carbone et pouvant comporter un ou plusieurs substituants choisis parmi les atomes d'halogènes, ou
  - un radical alcoxy renfermant au maximum 4 atomes de carbone.
- $R^3$ représente :
  - un atome d'hydrogène ou
  - un radical alkyle, linéaire ou ramifié, renfermant au maximum 12 atomes de carbone et pouvant comporter un ou plusieurs substituants choisis parmi les atomes d'halogènes.
- $R^1$ , $R^2$ et $R^3$ pouvant par ailleurs être identiques ou différents.

Plus spécifiquement $R^1$ et $R^2$, identiques ou différents, représentent :

- un atome d'hydrogène,
- un groupement hydroxyle,
- un groupement nitro, ou
- un radical alkyle, linéaire ou ramifié, qui renferme au maximum 4 atomes de carbone et dont les atomes d'hydrogène peuvent être partiellement ou totalement remplacés par des atomes de fluor, de chlore et/ou de brome

et, de préférence, lorsque l'un d'entre eux représente un groupement hydroxyle ou nitro, l'autre est choisi parmi l'hydrogène et les radicaux alkyles comportant au maximum 4 atomes de carbone.

A titre d'exemples de composés nitroaromatiques susceptibles d'être mis en oeuvre dans le cadre de la présente invention on peut citer :

- le nitrobenzène
- le p-nitrotoluène
- l'o-nitrotoluène
- le dinitro-2,4 toluène
- le p-nitroanisole
- le p-nitrophénol
- le p-nitrochlorobenzène et
- le p-(trifluorométhyl)nitrobenzène

Le procédé requiert la présence à titre de réactif (ou de matière première) d'un acide carboxylique de formule $R^3COOH$, dans laquelle $R^3$ à la signification précédemment donnée.

Plus spécifiquement $R_3$ représente un atome d'hydrogène ou un radical alkyle renfermant au maximum 4 atomes de carbone.

A titre d'exemples d'acides carboxyliques utilisables dans le cadre du présent procédé on peut citer : l'acide formique, l'acide acétique et l'acide propionique.

L'équation stoechiométrique proposée ci-avant fait apparaître des quantités équimolaires de composé nitroaromatique et d'acide carboxylique. La quantité d'acide carboxylique à mettre en oeuvre peut s'écarter largement de cette proportion 1/1 (molaire) et la quantité précise d'acide à mettre en oeuvre sera généralement supérieure à cette proportion. L'excès d'acide carboxylique qu'il s'avère souhaitable d'engager à la réaction dépendra pour une large mesure de la nature précise de l'acide carboxylique choisi et des divers autres paramètres qui influent sur le bon déroulement de la réaction.

Ainsi, par exemple, lorsqu'on utilise de l'acide formique, de bons résultats peuvent être observés avec un rapport molaire acide/composé nitroaromatique compris entre 1 et 10 et, de préférence, entre environ 2 et 4.

Par contre, lorsqu'on utilise par exemple de l'acide acétique de bons résultats peuvent être encore observés avec un rapport molaire largement supérieur à 10.

La mise en oeuvre du procédé selon la présente invention requiert la présence d'une quantité catalytiquement efficace de palladium ou d'un composé du palladium.

N'importe qu'elle source de palladium est susceptible de convenir à cette fin.

En effet, on peut recourir à du palladium métallique, pris tel quel ou déposé sur un support inerte tel le noir de carbone ou l'alumine, à des sels ou complexes du palladium.

Dans les conditions de la réaction en cause la majorité de ces sources de palladium sera soluble dans le milieu réactionnel. A titre d'exemples de composés du palladium convenant à la mise en oeuvre du présent procédé on peut citer :

- les carboxylates de palladium dont l'anion comporte, de préférence au maximum 12 atomes de carbone et en particulier l'acétate de palladium et le propionate de palladium ;
- les halogénures de palladium et en particulier la chlorure de palladium et le bromure de palladium ;
- l'acétylacétonate de palladium, les complexes du palladium et de la dibenzylidèneacétone tel Pd-$(dba)_3$.

L'acétate de palladium convient particulièrement bien à la mise en oeuvre du procédé selon l'invention.

La quantité précise de palladium à mettre en oeuvre dans le cadre du présent procédé peut varier dans de larges limites et résultera généralement d'un compromis entre l'efficacité souhaitée et la dépense en catalyseur et, des autres conditions choisies pour la réaction.

Un rapport molaire groupes $NO_2$/Pd compris entre 1000 et 200 paraît généralement satisfaisant ; un rapport supérieur à 1000 est susceptible de limiter la vitesse de réaction et un rapport inférieur à 200 risque de grever l'économie globale d'un tel procédé.

La mise en oeuvre du présent procédé requiert également la présence d'au moins un additif de formule rappelée ci-après dans laquelle G et G' ont la signification indiquée précédemment :

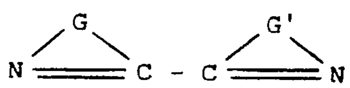

Lorsque le(s) groupe(s) pontant(s) G et/ou G' comporte(nt) un(ou deux) atome(s) distinct(s) des atomes de carbone, ces atomes sont de préférence des atomes d'azote. G et G' peuvent en outre être reliés entre eux par un groupe de deux atomes de carbone pour former un additif de formule ci-avant présentant le squelette de la phénantroline-1,10.

A titre d'exemples d'additifs de formule générale ci-avant on peut citer : le bipyridyle-2,2' le diméthyl-4,4' bipyridyle-2,2', le diméthoxy-4,4' bipyridyle-2,2', le dicarboxy-4,4' bipyridyle-2,2', le dichloro-4,4' bipyridyle-2,2', le biquinolyle-2,2', la phénanthroline-1,10, la diphényl-4,7 phénanthroline-1,10, la tétraméthyl-3,4,7,8 phénanthroline-1,10, et la diméthyl-4,7 phénanthroline-1,10. La phénanthroline-1,10 et ses dérivés non substitués sur les positions 2 et/ou 9 conviennent plus particulièrement comme additifs dans le cadre du présent procédé.

La quantité d'additif peut varier dans de larges limites. Elle est généralement telle que le rapport molaire additif/Pd soit compris entre 1 et 100 et, de préférence, entre 4 et 20.

La température de réaction dépend notamment de la réactivité du composé nitroaromatique. Une température d'au moins 110 °C paraît nécessaire pour obtenir une conversion acceptable et il n'y a pas d'avantage à opérer au delà de 180 °C, température à laquelle on peut observer une diminution sensible de la sélectivité en amide recherché. De préférence, la température est comprise entre 120 et 160 °C.

La réaction est conduite en phase liquide sous pression supérieure à la pression atmosphérique. Une pression partielle de monoxyde de carbone en température de l'ordre de 30 bar (3000 KPa) a un effet déjà sensible sur le bon déroulement de la réaction. Il n'est pas utile de dépasser 150 bar (15000 KPa).

Cette pression est avantageusement comprise entre 30 et 120 bar (3000 et 6000 KPa). On utilise du monoxyde de carbone sensiblement pur tel qu'il se présente dans le commerce, des quantités mineures de gaz inertes tels l'azote, l'argon et le gaz carbonique étant parfaitement tolérables. La présence d'hydrogène même en quantités notables n'est pas nuisible au déroulement de la réaction mais elle est susceptible d'induire une diminution sensible quoique faible de la sélectivité en produits recherchés.

Bien entendu la réaction en cause peut être conduite en présence d'un solvant ou diluant inerte vis à vis des produits de la réaction et de la réaction . A titre d'exemples de tels solvants ou diluants on peut citer : les hydrocarbures aromatiques chlorés ou non en particulier, le benzène et l'o-dichlororobenzène, les éthers aliphatiques ou cycliques, les amides N,N-disubstitués, les sulfones et les esters.

Certains acides carboxyliques (réactifs) tel, notamment, l'acide acétique pourront être engagés en large excès par rapport à la stoechiométrie évoquée en tête du présent mémoire et jouer en quelque sorte le rôle supplémentaire de solvant ou diluant.

En fin de la durée impartie à la réaction on sépare l'amide recherché du milieu réactionnel par tout moyen approprié par exemple par extraction et/ou distillation.

Les exemples ci-après illustrent la présente invention.

EXEMPLES :

Exemple 1 à 8 : Préparation du N-phénylformamide

Mode opératoire :

Le mode opératoire est décrit par référence à l'exemple 2 ci-après. Dans un autoclave en Hastelloy B 2 de 125 ml, on introduit 36 mmol de nitrobenzène ($ONO_2$), 0,36 matg de palladium sous forme de $Pd(OAc)_2$, 3 mmol de phénanthroline-1,10 (phén.), 72 mmol d'acide formique et de l'O-dichlororobenzène qsp 30 ml (odcb).

L'autoclave est purgé par de l'oxyde de carbone puis porté sous agitation à 140° C, sous 60 bar (6000 KPa) de CO, pression maintenue constante. Après 1 h de réaction à la température indiquée, l'autoclave est refroidi et et dégazé. La masse réactionnelle est soutirée, diluée à l'éthanol (qsp 100 ml) et analysée par chromatographie en phase gazeuse.

Les exemples 1, 3 à 8 sont réalisés selon un mode opératoire analogue. Les conditions particulières et les résultats obtenus sont rassemblés dans le tableau (I) annexé dans lequel les charges initiales sont ramenées à 1 mol de nitrobenzène et les conventions suivantes sont utilisées :

| | |
|---|---|
| T : | désigne la température de réaction en ° C |
| t : | désigne la durée de réaction en heure |
| P : | désigne la pression de CO, en température, en bar |
| TT % : | désigne le taux de transformation du nitrobenzène. |
| RT % (NPF) : | désigne le nombre de moles de N-phénylformamide formées pour 100 moles de nitrobenzène transformées. |
| RT % (A) : | désigne le nombre de moles d'aniline formées pour 100 moles de nitrobenzène transformées. |

Exemple 9 :

On reproduit l'exemple 2 ci-avant en remplaçant la phénanthroline-1,10 par une quantité équivalente de bipyridyle-2,2'.

Les résultats obtenus sont les suivants :

| | |
|---|---|
| TT % : | 59 |
| RT % (NPF) : | 79 |
| RT % (A) : | 2 |

Exemples 10 à 12 : Préparation de divers N-arylformamides (NAF) :

Dans l'appareillage défini ci-avant et selon un mode opératoire analogue à celui décrit précédemment on réalise une série d'essais à 140° C sous 60 bar au départ de 3 composés nitroaromatiques distincts.

L'exemple 10 est réalisé au départ de l'o-nitrotoluène ; l'exemple 11 est réalisé au départ de dinitro-2,4 toluène et l'exemple 13, au départ de (trifluorométhyl-4 nitrobenzène).

En fin de chaque essai, la masse réactionnelle soutirée est évaporée puis distillée boule à boule. Le distillat est analysé par chromatographie en phase gazeuse, par couplage CPG/Spectroscopie de Masses et RMN.

Les conditions particulières ainsi que les résultats obtenus sont rassemblés dans le tableau (II) annexé dans lequel les charges initiales sont ramenées à 1 mol de composé nitroaromatique et dans lequel :

| | |
|---|---|
| TT % : | désigne le taux de transformation du composé nitroaromatique en cause |
| RT % (NAF) : | désigne le nombre de moles de N-arylformamide formées pour 100 moles de composé nitroaromatique transformées. |

Exemples 13 et 14 : Préparation de l'acétanilide et du p-acétylaminophénol

Dans l'appareillage défini ci-avant et selon un mode opératoire analogue à celui décrit ci-avant on réalise successivement deux synthèses à 140 °C sous 60 bar en l'absence toutefois d'O-dichlorobenzène.

L'analyse après synthèse de l'acétanilide est réalisée comme indiqué pour les exemples 1 à 8 ci-avant ; celle correspondant à la synthèse du p-acétylaminophénol est réalisée par CLHP sur la masse réactionnelle soutirée et évaporée au préalable.

Les conditions particulières ainsi que les résultats obtenus figurent dans le tableau III annexé.

TABLEAU I

| Réf | CHARGES INITIALES | | | | | COND. OPE. | | | TT% | RT%(NPF) | RT%(A) |
|---|---|---|---|---|---|---|---|---|---|---|---|
| | mol | | | | ml | | | | | | |
| | ØNO2 | HCOOH | Pd | phen. | odcb | T°C | t | P | | | |
| 1 | 1 | 20 | 0,01 | 0,085 | - | 140 | 3 | 60 | 10 | 42 | 0 |
| 2 | 1 | 2 | 0,01 | 0,085 | 640 | 140 | 1 | 60 | 65 | 86 | 0 |
| 3 | 1 | 1,2 | 0,01 | 0,085 | 700 | 140 | 2 | 60 | 40 | 83 | 0,5 |
| 4 | 1 | 10 | 0,01 | 0,085 | 280 | 140 | 2 | 60 | 9 | 38 | 2 |
| 5 | 1 | 2 | 0,01 | 0,085 | 650 | 140 | 1 | 30 | 38 | 84 | 0 |
| 6 | 1 | 2 | 0,01 | 0,085 | 650 | 110 | 1 | 60 | 18 | 84 | 2 |
| 7 | 1 | 2 | 0,01 | 0,085 | 650 | 180 | 1 | 60 | 84 | 81 | 1 |
| 8 | 1 | 2 | 0,005 | 0,085 | 650 | 140 | 1 | 60 | 54 | 89 | 0 |

TALEAU II

---------

| Réf | COMP. NITRO-AROMATIQUE | | HCOOH mol | Pd atg | Phen. mol | odcb ml | t | TT% | RT%(NAF) |
|---|---|---|---|---|---|---|---|---|---|
| | Nature | Mol | | | | | | | |
| 10 | 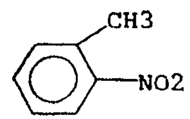 | 1 | 2 | 0,01 | 0,09 | 640 | 2 | 50 | 41 |
| 11 | 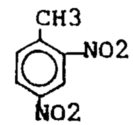 | 1 | 4 | 0,01 | 0,09 | 560 | 2 | 24 | N.B. |
| 12 | 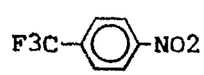 | 1 | 2 | 0,01 | 0,09 | 640 | 1 | 100 | 78 |

N.B. La formation des deux formamides suivants, est observée :

- 28 % 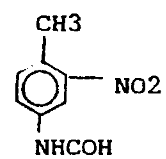

- 24 % 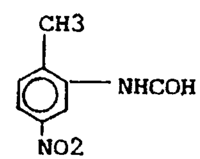

TABLEAU III

| Réf | COMP. NITRO-AROMATIQUE Nature | Mol | $H_3COOH$ mol | Pd atg | phen. mol | P | T°C | t | TT(*) % | RT(**) % |
| --- | --- | --- | --- | --- | --- | --- | --- | --- | --- | --- |
| 13 | 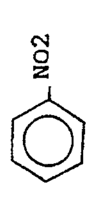 | 1 | 13 | 0,008 | 0,08 | 60 | 140 | 3,5 | 100 | 92 |
| 14 | 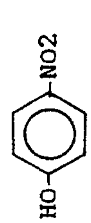 | 1 | 13 | 0,008 | 0,08 | 60 | 140 | 3 | 98 | 61 |

(*) du composé nitroaromatique

(**) respectivement en anilide et en p-acétylaminophénol

## Revendications

1. Procédé de préparation d'amides N-arylsubstitués par réaction en phase liquide, sous pression supérieure à la pression atmosphérique, de l'oxyde de carbone et d'au moins un acide carboxylique

sur au moins un composé nitroaromatique caractérisé en ce que la réaction est conduite en présence d'une quantité catalytiquement efficace de palladium ou d'un composé du palladium et d'au moins un additif de formule :

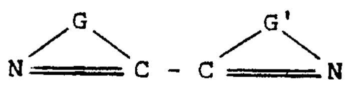

dans laquelle G et G', identiques ou différents représentent chacun un groupe pontant qui comporte 3 ou 4 atomes dont au moins deux sont des atomes de carbone, G et G' pouvant par ailleurs être reliés l'un à l'autre.

**2.** Procédé selon la revendication 1, caractérisé en ce que la température de réaction est supérieure ou égale à 110 °C et est inférieure ou égale à 180 °C.

**3.** Procédé selon la revendication 1 ou 2, caractérisé en ce que la pression partielle de l'oxyde de carbone mesurée en température est supérieure ou égale à 30 bar (3000 KPa).

**4.** Procédé selon l'une quelconque des revendications précédentes, caractérisé en ce que la pression partielle de l'oxyde de carbone mesurée en température est inférieure ou égale à 120 bar (12000 KPa).

**5.** Procédé selon l'une quelconque des revendications précédentes, caractérisé en ce que le composé aromatique est choisi parmi le nitrobenzène, les nitrotoluènes, les dinitrotoluènes, le p-(trifluorométhyl)-nitrobenzène et le p-nitrophénol.

**6.** Procédé selon l'une quelconque des revendications précédentes, caractérisé en ce que l'acide carboxylique est l'acide acétique.

**7.** Procédé selon l'une quelconque des revendications 1 à 5, caractérisé en ce que l'acide carboxylique est l'acide formique.

**8.** Procédé selon l'une quelconque des revendications 1 à 6, caractérisé en ce que le composé nitroaromatique est le p-nitrophénol.

**9.** Procédé selon l'une quelconque des revendications précédentes, caractérisé en ce que l'additif est choisi dans le groupe comprenant le bipyridyle-2,2' le diméthyl-4,4' bipyridyle-2,2', le diméthoxy-4,4' bipyridyle-2,2', le dicarboxy-4,4' bipyridyle-2,2', le dichloro-4,4' bipyridyle-2,2', le biquinolyle-2,2', la phénanthroline-1,10, la diphényl-4,7 phénanthroline-1,10 la tétraméthyl-3,4,7,8 phénanthroline-1,10, et la diméthyl-4,7 phénanthroline-1,10.

**10.** Procédé selon la revendication 9 caractérisé en ce que l'additif est la phénanthroline-1,10.

**11.** Procédé selon l'une quelconque des revendications précédentes, caractérisé en ce que le rapport molaire groupe $NO_2$/Pd est compris entre 1000 et 200.

**12.** Procédé selon l'une quelconque des revendications précédentes, caractérisé en ce que le rapport molaire additif/Pd est compris entre 1 et 100.

Office européen des brevets

# RAPPORT DE RECHERCHE EUROPEENNE

Numéro de la demande

**EP 90 40 1251**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | THE JOURNAL OF ORGANIC CHEMISTRY, vol. 49, no. 23, 16 novembre 1984, pages 4451-4455, American Chemical Society, US; Y. WATANABE et al.: "Platinum complex catalyzed reductive N-acylation of nitro compounds" * Tableaux I,II * | 1,5 | C 07 C 231/10<br>C 07 C 233/25<br>C 07 C 233/15 |
| A,D | BULLETIN OF THE CHEMICAL SOCIETY OF JAPAN, vol. 42, no. 3, mars 1969, pages 827-828, Tokyo, JP; T. KAJIMOTO et al.: "Organic syntheses by means of metal complexes. IV. Reduction of nitrobenzene with carbon monoxide" * Tableau I * | 1,5 | |
| A | EP-A-0 293 999 (SHELL) * Revendications * | 1,5 | |
| E | FR-A-2 644 161 (RHONE-POULENC CHIMIE) * Revendications * | 1-12 | |

DOMAINES TECHNIQUES RECHERCHES (Int. Cl.5)

C 07 C 231/00
C 07 C 233/00

Le présent rapport de recherche a été établi pour toutes les revendications

| Lieu de la recherche | Date d'achèvement de la recherche | Examinateur |
|---|---|---|
| La Haye | 09 janvier 91 | SANCHEZ Y GARCIA J.M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

&: membre de la même famille, document correspondant